# EUROPÄISCHE PATENTSCHRIFT

(11) **EP 2 758 785 B1**
(45) Veröffentlichungstag und Bekanntmachung des Hinweises auf die Patenterteilung: **23.01.2019**
(21) Anmeldenummer: 12772206.4
(22) Anmeldetag: 19.09.2012
(51) Int. Cl.: G01N 33/569

(54) **VERFAHREN ZUM ÜBERPRÜFEN DER SELBSTHEILUNG DURCH DAS IMMUNSYSTEM EINES MIT HUMANEN PAPILLOMVIREN INFIZIERTEN PROBANDEN**
METHOD FOR CHECKING THE SELF-HEALING PROCESS CARRIED OUT BY THE IMMUNE SYSTEM OF A TEST SUBJECT INFECTED WITH HUMAN PAPILLOMAVIRUS
PROCÉDÉ DE CONTRÔLE DE L'AUTO-GUÉRISON PAR LE SYSTÈME IMMUNITAIRE D'UN SUJET D'EXPÉRIENCE INFECTÉ PAR LE VIRUS DU PAPILLOME HUMAIN

(30) Priorität: 19.09.2011 DE 102011053741
(43) Veröffentlichungstag der Anmeldung: 30.07.2014
(73) Patentinhaber: Abviris Deutschland GmbH, 22926 Ahrensburg (DE)
(72) Erfinder: HILFRICH, Ralf, 65597 Hünfelden (DE)
(74) Vertreter: Launhardt, Thomas
(86) Internationale Anmeldenummer: PCT/DE2012/100287
(87) Internationale Veröffentlichungsnummer: WO 2013/041087

(56) Entgegenhaltungen:
- EP-A1- 1 271 151
- WO-A2-2007/059492
- WO-A2-2009/042488
- AALTONEN L M ET AL: "POOR ANTIBODY RESPONSE AGAINST HUMAN PAILLOMAVIRUS IN ADULT-ONSET LARYNGEAL PAPILLOMATOSIS", JOURNAL OF MEDICAL MICROBIOLOGY, HARLOW, GB, Bd. 50, Nr. 5, 1. Mai 2001 (2001-05-01), Seiten 468-471, XP001083684, ISSN: 0022-2615
- HILFRICH RALF ET AL: "Prognostic relevance of human papillomavirus L1 capsid protein detection within mild and moderate dysplastic lesions of the cervix uteri in combination with p16 biomarker", ANALYTICAL AND QUANTITATIVE CYTOLOGY AND HISTOLOGY, ST. LOUIS, MO, US, Bd. 30, Nr. 2, 1. April 2008 (2008-04-01), Seiten 78-82, XP009165575, ISSN: 0884-6812
- GRIESSER HENRIK ET AL: "HPV Vaccine Protein L1 Predicts Disease Outcome of High-Risk HPV plus Early Squamous Dysplastic Lesions", AMERICAN JOURNAL OF CLINICAL PATHOLOGY, AMERICAN SOCIETY FOR CLINICAL PATHOLOGY, US, Bd. 132, Nr. 6, 1. Dezember 2009 (2009-12-01), Seiten 840-845, XP009165577, ISSN: 0002-9173
- SCHEIDEMANTEL THOMAS ET AL: "Expression Pattern of HPV L1 Capsid Protein in PAP Tests: A Potential Biomarker in Risk Assessment for High Grade SIL Lesion", CANCER, AMERICAN CANCER SOCIETY, PHILADELPHIA, PA, US, Bd. 114, Nr. 5, Suppl. S, 7. November 2008 (2008-11-07), Seite 349, XP009165576, ISSN: 0008-543X
- HILFRICH R ET AL: "Prognostic Significance of HPV L1 Capsid Protein Detection with Cytoactiv (R) in an International Multicenter Study of 3000 Early Dysplastic Lesions", LABORATORY INVESTIGATION, NATURE PUBLISHING GROUP, THE UNITED STATES AND CANADIAN ACADEMY OF PALHOLOGY, INC, Bd. 90, Nr. Suppl. 1, 26. März 2010 (2010-03-26), Seite 94A, XP009165579, ISSN: 0023-6837
- HEIM KURT ET AL: "Antibodies to human papillomavirus 16 L1 virus-like particles as an independent prognostic marker in cervical cancer", AMERICAN JOURNAL OF OBSTETRICS AND GYNECOLOGY, Bd. 186, Nr. 4, April 2002 (2002-04), Seiten 705-711, XP002689205, ISSN: 0002-9378
- T. D. D. Gruijl ET AL: "Immunoglobulin G Responses Against Human Papillomavirus Type 16 Virus-Like Particles in a Prospective Nonintervention Cohort Study of Women With Cervical Intraepithelial Neoplasia", JOURNAL OF THE NATIONAL CANCER INSTITUTE, vol. 89, no. 9, 7 May 1997 (1997-05-07), pages 630-638, XP055263824, GB ISSN: 0027-8874, DOI: 10.1093/jnci/89.9.630
- RAUBER D ET AL: "Prognostic significance of the detection of human papilloma virus L1 protein in smears of mild to moderate cervical intraepithelial lesions", EUROPEAN JOURNAL OF OBSTETRICS & GYNECOLOGY AND REPRODUCTIVE BIOLOGY, ELSEVIER IRELAND LTD, IE, vol. 140, no. 2, 1 October 2008 (2008-10-01), pages 258-262, XP025471238, ISSN: 0301-2115, DOI: 10.1016/J.EJOGRB.2008.05.003 [retrieved on 2008-07-14]

## Beschreibung

Die Erfindung betrifft ein Verfahren zur Vorhersage der Bildung von Karzinomen, welche durch humane Papillomviren bedingt sind, bei einem mit den humanen Papillomviren infizierten Probanden.

Humane Papillomviren, abgekürzt HPV und nachfolgend auch HPV genannt, bilden eine Gruppe von Viren, die mittlerweile in mehr als 100 Typen eingeteilt werden. Die Viren befallen die Epithelzellen der Haut oder verschiedener Schleimhäute und verursachen bei den infizierten Zellen ein unkontrolliertes, tumorartiges Wachstum.

Einige HPV-Typen können bösartige Veränderungen hervorrufen. Beispielsweise stehen humane Papillomviren in Verdacht, bei der Entstehung von Gebärmutterhalskrebs, dem sogenannten Zervixkarzinom, beteiligt zu sein. Auch stehen die humanen Papillomviren in Verdacht, Scheiden-, Penis- und Analkarzinome zu verursachen oder zumindest an deren Entstehung beteiligt zu sein.

Untersuchungen an HPV-Infizierten haben ergeben, dass eine Infektion mit Karzinom verursachenden humanen Papillomviren noch lange nicht zu einer Bildung von Karzinomen führt. Die Untersuchungen haben gezeigt, dass in vielen Fällen das Immunsystem auf die HPV-Infizierung reagiert und Maßnahmen zur Zerstörung der HPV einleitet. Beispielsweise werden von dem Immunsystem sogenannte Killerzellen aktiviert, welche die humanen Papillomviren frühzeitig zerstören, so dass einer Karzinombildung wirkungsvoll entgegengewirkt ist. Eine mit HPV infizierte Person wird in diesem Fall mit sehr hoher Wahrscheinlichkeit nicht an einem durch HPV hervorgerufenen Karzinom erkranken.

Sofern das Immunsystem jedoch nicht auf eine solche HPV-Infizierung reagiert oder zumindest nicht mit den entsprechenden Maßnahmen auf die HPV-Infizierung reagiert, kommt es mit hoher Wahrscheinlichkeit zur Karzinombildung.

Heim et al: "Antibodies to human papillomvirus 16 L1 virus-like particles as an independent prognostic marker in cervical cancer", American Journal of Obstetrics and Gynecology, Bd. 186, Nr. 4, April 2002 (2002-04), Seiten 705-711 haben aufgezeigt, dass das Vorhandensein von körpereigenen Antikörpern gegen HPV einen positiven Einfluss bei solchen Patienten hat, welche bereits HPV bedingte Karzinome aufweisen. Es wurde beobachtet, dass Patienten mit HPV bedingten Karzinomen länger lebten, wenn körpereigene Antikörper gegen die HPV bei den Patienten nachgewiesen wurden.

Aaltonen et al: "Poor antibody response against human papillomavirus in adult-onset laryngeal papillomatosis", Journal of Medical Microbiology, Harlow, GB, Bd. 50, Nr. 5, 1. Mai 2001, Seiten 468-471 haben Patienten mit Warzen im Rachen untersucht, welche durch HPV vom Typ 6 und/oder HPV vom Typ 11 verursacht sind. Bei diesen Patienten wurden körpereigene Antikörper nachgewiesen, wobei diese Antikörper auch bei einer Kontrollgruppe von Patienten nachgewiesen werden konnten, welche keine Warzen im Rachen aufwiesen. Daraus wurde gefolgert, dass die nachgewiesenen körpereigenen Antikörper keine Reaktion des Immunsystems auf die Bildung der Warzen sein können.

Gruijl et al: "Immunoglobulin G responses against human papillomavirus type 16 virus-like particles in a prospective nonintervention cohort study of women with cervical intraepithelial neoplasia", Journal of the National Cancer Institute, Bd. 89, Nr. 9, 7. Mai 1997, Seiten 630-638 haben eine Gruppe von Frauen untersucht, welche mit HPV infiziert waren und bereits HPV bedingte Krebsvorstufen aufwiesen. Es wurde beobachtet, dass die Frauen, wenn körpereigene Antikörper gegen die HPV nachgewiesen wurden, tendenziell ein progressives Fortschreiten der HPV bedingten Erkrankung zeigten.

Der Erfindung liegt die Aufgabe zugrunde, eine Methode vorzuschlagen, mittels welcher eine belastbare Vorhersage besser als bisher möglich ist, ob es bei mit humanen Papillomviren Infizierten auch tatsächlich zu einer Bildung von Karzinomen kommt.

Die Aufgabe wird mit einem Verfahren gelöst, welches die Merkmale des Anspruches 1 aufweist. Vorteilhafte Weiterbildungen der Erfindung ergeben sich aus den Unteransprüchen, der nachfolgenden Beschreibung und den Figuren.

Ein erfindungsgemäßes Verfahren ist zum Überprüfen der Selbstheilung durch das Immunsystem eines mit humanen Papillomviren infizierten Probanden anhand wenigstens zweier von dem Probanden entnommenen, Zellen enthaltenden Proben geeignet. Das Verfahren umfasst folgende Schritte:
1. Feststellung, ob HPV mit wenigstens einem das Immunsystem des Probanden stimulierenden HPV-spezifischen Stimulusmolekül in wenigstens einer der Proben enthalten sind;
2. Feststellung, ob körpereigene Antikörper gegen die HPV mit dem Stimulusmolekül in wenigstens einer anderen der Proben enthalten sind;
3. Auswertung der Schritte 1. und 2. dahingehend, dass bei Vorliegen des Stimulusmoleküls und bei Vorliegen der Antikörper die Selbstheilung durch das Immunsystem des Probanden in Gang gesetzt ist, welche die Bildung eines HPV-induzierten Karzinoms verhindert.

Die Erfindung beruht auf dem Gedanken, zur Überprüfung der Selbstheilung durch das Immunsystem eines mit HPV Infizierten zwei Bestimmungsgrößen zugrunde zu legen, nämlich zum einen, ob der HPV-Infizierte ein sein Immunsystem stimulierendes HPV-spezifisches Stimulusmolekül hat und zum anderen, ob der HPV-Infizierte bereits körpereigene Antikörper gebildet hat, welche gegen die HPV mit dem Stimulusmolekül wirken.

Es hat sich gezeigt, dass durch das erfindungsgemäße Verfahren eine Methode realisiert ist, mittels welcher mit großer Sicherheit vorhergesagt wird, ob es bei mit HPV infizierten Probanden zu einer HPV bedingten Ausbildung von Karzinomen kommt oder ob durch die Abwehrkräfte des Immunsystems eine solche Karzinombildung verhindert wird.

Das erfindungsgemäße Verfahren ist zur Anwendung bei Lebewesen aller Art geeignet. Grundsätzlich kann das erfindungsgemäße Verfahren daher sowohl bei Menschen als auch bei Tieren eingesetzt werden.

Das erfindungsgemäße Verfahren eignet sich besonders zur Überprüfung der Selbstheilung durch das Immunsystem einer Frau, deren Gebärmutter, insbesondere deren Gebärmutterhals mit HPV infiziert ist. Mittels des erfindungsgemäßen Verfahrens lässt sich eine belastbare Vorhersage treffen, ob sich im Bereich der Gebärmutter, insbesondere des Gebärmutterhalses, HPV-verursachte bösartige Tumore bilden werden. Insofern ist das erfindungsgemäße Verfahren eine besonders gute Hilfe bei der Entscheidung, ob an der Frau ein operativer Eingriff vorzunehmen ist, beispielsweise um eine Konisation auszuführen.

Das erfindungsgemäße Verfahren kann wie folgt ausgeführt werden: Es wird anhand einer entnommenen Probe zuerst geprüft, ob wenigstens eine der mit der Probe entnommenen Zellen mit HPV infiziert ist, welche das Stimulusmolekül aufweist. Sofern die Prüfung positiv ausfällt, also HPV mit dem Stimulusmolekül festgestellt werden, wird in dem sich anschließenden Schritt geprüft, ob das Immunsystem des Probanden auf die vorliegende HPV-Infizierung bereits reagiert hat, indem festgestellt wird, ob das Immunsystem bereits körpereigene Antikörper gegen die HPV mit dem Stimulusmolekül gebildet hat.

Zur Überprüfung, ob körpereigene Antikörper gebildet worden sind, wird wenigstens eine weitere Probe von dem Probanden entnommen, beispielsweise zu einem späteren Zeitpunkt. Diese Überprüfung kann beispielsweise auch anhand einer Probe durchgeführt werden, welche an einer anderen Stelle des Probanden als die vorhergehende Probe entnommen wurde. Grundsätzlich werden zur Durchführung der Überprüfung, ob körpereigene Antikörper gebildet worden sind, Körperflüssigkeiten aller Art verwendet, wie beispielsweise Blut, Serum oder Plasma.

Das erfindungsgemäße Verfahren ist auf wenigstens einen Typ eines Kapsids der HPV als Stimulusmolekül ausgerichtet. Als Stimulusmolekül ist das L1-Protein des Kapsids der HPV vom Typ 16 herangezogen.

Bisherige Untersuchungen haben gezeigt, dass bei HPV-Infizierten, deren Zellen das L1-Protein noch bilden, mit einer Wahrscheinlichkeit von über 80% eine Bildung von HPV-bedingten Karzinomen ausbleibt. Wenn das Immunsystem des Infizierten körpereigene Antikörper gegen die L1-Proteine gebildet hat und auf diese Weise auf die HPV-Infektion reagierte, blieben bisher Karzinome durchgängig aus.

Die Untersuchungen haben ferner aufgezeigt, dass es mit einer Wahrscheinlichkeit von über 90% bei den HPV-Infizierten zu einer HPV-bedingten Bildung eines Karzinoms kommt, sofern der L1-Stimulus fehlt und das Immunsystem keine körpereigenen Antikörper gegen das L1-Protein bildet.

Vor diesem Hintergrund bietet es sich an, dass erfindungsgemäß zuerst festgestellt wird, ob HPV mit wenigstens einem L1-Protein als Stimulusmolekül in der Probe enthalten sind und anschließend, sofern HPV mit dem L1-Protein festgestellt werden, überprüft wird, ob die Bildung von körpereigenen Antikörpern gegen das L1-Protein bei dem Probanden eingesetzt hat.

Die Entnahme der Probe erfolgt von der Schleimhaut des Probanden. Insofern bietet es sich an, dass zur Probennahme ein Abstrich von einer Schleimhautoberfläche des Probanden, beispielsweise ein Abstrich von der Schleimhaut des Gebärmutterhalses einer Frau, vorgenommen wird.

Zur Feststellung, ob HPV mit dem Stimulusmolekül in der wenigstens einen Probe enthalten sind, wird ein Schritt ausgeführt, in dem ein Erkennen einer morphologischen Veränderung und/oder molekularbiologischen Veränderung wenigstens einer der Zellen, vorzugsweise gegenüber einem Referenzzustand, erfolgt. Dadurch werden gesunde Probanden erkannt, da deren Zellen eine morphologische Veränderung bzw. molekularbiologische Veränderung nicht aufweisen. In diesem Fall ist eine weitere Durchführung des erfindungsgemäßen Verfahrens nicht notwendig, so dass die weiteren Schritte des erfindungsgemäßen Verfahrens eingespart werden können und somit sich eine Kosteneinsparung ergibt.

Um unterscheiden zu können, dass es sich bei einer ermittelten Veränderung um eine Abweichung gegenüber dem gesunden Zustand handelt, wird bevorzugt die morphologische Veränderung und/oder die molekularbiologische Veränderung durch einen Vergleich mit gesunden Zellen vorgenommen.

Zum Erkennen der morphologischen Veränderung und/oder molekularbiologischen Veränderung der wenigstens einen Zelle kann die Probe einer Analyse zugeführt werden, welche eine messbare und/oder von einem Benutzer wahrnehmbare Veränderung, vorzugsweise gegenüber einem Referenzzustand, nutzt, um daraus Rückschlüsse auf die morphologische bzw. molekularbiologische Veränderung der Zelle gegenüber dem gesunden Zustand der Zelle schließen zu können, insbesondere um damit eine solche Veränderung feststellen zu können.

Dazu kann eine mikroskopische Untersuchung der Probe vorgenommen werden, beispielsweise kann eine morphologische Veränderung der wenigstens einen Zelle, vorzugsweise gegenüber einem Referenzzustand, im Anschluss an eine Einfärbung der Probe unter dem Mikroskop festgestellt werden. Es bietet sich zum Erkennen der morphologischen Veränderung der wenigstens einen Zelle an, dass ein Pap-Test durchgeführt wird. Zum Erkennen der molekularbiologischen Veränderung der wenigstens einen Zelle ist es beispielsweise möglich, dass die Probe einer Analyse zugeführt wird, in welcher ein Mutieren der Zelle und/oder zusätzliche Erbinformationen, wie beispielsweise virale Erbinformationen, in der Zelle festgestellt werden kann.

Zur Feststellung, ob die HPV mit dem Stimulusmolekül in der wenigstens einen anderen der Proben enthalten sind, wird ein weiterer Schritt ausgeführt, in dem mittels HPV-spezifischer Antikörper die morphologische und/oder molekularbiologische Veränderung der wenigstens einen Zelle, vorzugsweise gegenüber einem Referenzzustand, als HPV-verursachte Veränderung identifiziert wird, an welcher HPV mit dem Stimulusmolekül beteiligt sind. Bereits durch diesen weiteren Schritt lässt sich eine erste Abschätzung vornehmen, ob es bei dem HPV-Infizierten zu der Bildung eines HPV-verursachten Karzinoms kommt.

Untersuchungen haben ergeben, dass beispielsweise Probanden mit einer HPV-Infizierung, bei denen das Stimulusmolekül nicht gebildet wird, also das Stimulusmolekül nicht festgestellt wird, mit hoher Wahrscheinlichkeit an einem Karzinom erkranken wird. Der Proband wird dagegen mit hoher Wahrscheinlichkeit keine HPV-verursachten Karzinome bilden, sofern das Vorhandensein des Stimulusmoleküls in der Probe mittels dieses Schrittes identifiziert wird. Für diesen Fall wird dann in dem anschließenden Schritt des erfindungsgemäßen Verfahrens festgestellt, ob das Immunsystem durch das Stimulusmolekül bereits angesprungen ist und körpereigene Antikörper gegen die HPV bzw. das Stimulusmolekül gebildet werden.

Der wenigstens eine HPV-spezifische Antikörper ist dazu ausgebildet, sich an wenigstens ein das Stimulusmolekül aufweisende HPV anzubinden, so dass bei Vorhandensein des Stimulusmoleküls über den Antikörper auf das damit verbundene Stimulusmolekül geschlossen werden kann.

Der HPV-spezifische Antikörper kann ein nicht-humaner Antikörper sein. Denkbar ist es ferner, dass der HPV-spezifische Antikörper ein humaner Antikörper ist. Auch kann der HPV-spezifische Antikörper ein körpereigener Antikörper sein.

Es bietet sich an, dass der HPV-spezifische Antikörper ein Mausantikörper, insbesondere ein monoklonaler Mausantikörper ist, da ein derartiger Antikörper in einfacher Weise gezielt für die Anwendung in dem erfindungsgemäßen Verfahren hergestellt werden kann.

Um als Stimulusmolekül ein Kapsid zu identifizieren, ist der Antikörper ein Kapsid-spezifischer Antikörper. Ferner ist der Antikörper ein L1-spezifischer Antikörper, nachfolgend auch als HPVL1-spezifischer Antikörper bezeichnet, um als Antikörper gegen das L1-Protein der HPV zu wirken.

Nach einer Ausgestaltung der Erfindung ist es vorgesehen, dass in dem weiteren Schritt die Probe zuerst wärmbehandelt wird, dann die HPV-spezifischen Antikörper zugegeben werden und anschließend die Probe mit den HPV-spezifischen Antikörpern einer Analyse zugeführt wird, welche eine messbare und/oder von einem Benutzer wahrnehmbare Veränderung der mit HPV infizierten Zellen, vorzugsweise gegenüber einem Referenzzustand, aufgrund der Zugabe der HPV-spezifischen Antikörper nutzt.

Durch die Wärmbehandlung werden bestehende Proteinstrukturen zerstört, wodurch die Anbindung der Antikörper an die mit HPV infizierten Zellen begünstigt wird. Durch die Wärmebehandlung erfolgt eine sogenannte Antigen-Demaskierung. Die Behandlung kann mittels Dampfhitze und/oder Mikrowellen ausgeführt werden. Bevorzugt erfolgt die Wärmebehandlung der Probe bei einer Temperatur zwischen etwa 90 Grad und etwa 100 Grad Celsius, vorzugsweise bei etwa 95 bis 96 Grad Celsius.

Es bietet sich an, dass ein Verstärkermittel zugegeben wird, durch welches die messbare und/oder von einem Benutzer wahrnehmbare Veränderung der mit HPV infizierten Zellen aufgrund der Zugabe der HPV-spezifischen Antikörper verstärkt wird. Durch das Verstärkermittel wird die Nachweisgrenze der Analyse, mittels welcher die mit HPV infizierten Zellen aufgrund der Zugabe der HPV-spezifischen Antikörper identifiziert werden, herabgesetzt. Insoweit lassen sich mittels des Verstärkermittels die HPV mit dem Stimulusmolekül und dem daran anhaftenden Antikörper besonders gut identifizieren.

Es bietet sich ferner an, dass das Verstärkermittel an weiteren HPV-spezifischen Antikörpern angebunden ist und zusammen mit den weiteren HPV-spezifischen Antikörpern zugegeben wird, wobei sich die weiteren HPV-spezifischen Antikörper an die HPV-infizierten Zellen und/oder die bereits zugegebenen HPV-spezifischen Antikörper anhaften. Dadurch ist in einfacher Weise ein Bindungskomplex erzeugt, welcher aus der mit HPV-infizierten Zelle, dem HPV-spezifischen Antikörper und dem weiteren HPV-spezifischen Antiköper mit dem Verstärkermittel bestehen.

Der weitere HPV-spezifische Antikörper kann ein nicht-humaner Antikörper sein. Denkbar ist es ferner, dass der weitere HPV-spezifische Antikörper ein humaner Antikörper ist. Auch kann der weitere HPV-spezifische Antikörper ein körpereigener Antikörper sein.

Es bietet sich an, dass der weitere HPV-spezifische Antikörper ein Mausantikörper, insbesondere ein monoklonaler Mausantikörper ist, da ein derartiger Antikörper in einfacher Weise gezielt für die Anwendung in dem erfindungsgemäßen Verfahren hergestellt werden kann.

Der weitere HPV-spezifische Antikörper ist natürlich ein solcher Antikörper, welcher auf das Stimulusmolekül der HPV reagiert und/oder auf den HPV-spezifischen Antikörper reagiert, welcher bereits an der Zelle mit den HPV und dem wenigstens einen Stimulusmolekül haftet.

Das Verstärkermittel kann ein als Farbverstärker wirkendes Molekül sein. Das Verstärkungsmittel kann ein Enzym, wie beispielsweise eine Peroxidase oder Phosphatase sein.

Sofern das Verstärkermittel ein als Farbverstärker wirkendes Molekül ist, bietet es sich an, dass die Analyse durch Zugabe einer vorgegebenen Menge an Farbstoff, wie beispielsweise eines Chromogens, zur Färbung der mit HPV-infizierten wenigstens einen Zelle, insbesondere des wenigstens einen daran haftenden HPV-spezifischen Antikörpers, erfolgt. Es ist dadurch in technischer einfacher Weise das HPV mit dem Stimulusmolekül zu identifzieren, da die HPV-infizierte Zelle und/oder der daran anhaftende Antikörper durch Färbung markiert sind.

Ergänzend oder alternativ kann eine Analyse bzw. Identifizierung der HPV-infizierten Zellen oder der daran anhaftenden HPV-spezifischen Antikörper vorgenommen werden, indem eine radioaktive Markierung vorgenommen wird und/oder die HPV-infizierten Zellen bzw. die daran haftenden HPV-spezifischen Antikörper durch Markierung mittels fluoreszierender Teilchen identifiziert wird.

Bevorzugt sollten die nicht an den HPV-infizierten Zellen haftenden Antikörper ausgewaschen werden. Dadurch ist sichergestellt, dass die nicht an den HPV-infizierten Zellen anhaftenden HPV spezifischen Antikörper aus der Probe herausgewaschen werden und nicht zur Analyse gelangen, so dass beispielsweise im Zuge einer Anfärbung der HPV-spezifischen Antikörper ausschließlich die an den HPV-infizierten haftenden Antikörper sichtbar gemacht werden.

Sofern zusätzlich zu den HPV-spezifischen Antikörpern auch die weiteren HPV-spezifischen Antikörper zugegeben werden, sollte nach jeder Zugabe ein Auswaschungsschritt erfolgen oder am Ende zumindest des letzten Zugabeschrittes eine Auswaschung erfolgen. Dies ebenfalls vor dem Hintergrund, lediglich die tatsächlich an den HPV-infizierten Zellen anhaftenden Antikörper in der Probe zu haben, um mittels deren Markierung, insbesondere Farb- oder Fluoreszenzmarkierung oder Markierungen mittels Radioaktivität, die HPV-infizierten Zellen mit dem Stimulusmolekül im Zuge der Analyse zu identifizieren.

Nach der Erfindung ist es vorgesehen, dass zur Feststellung, ob körpereigene Antikörper gegen die HPV mit dem Stimulusmolekül vorhanden sind, ein Test zum qualitativen und/oder quantitativen Bestimmen von in Körperflüssigkeit enthaltenen Antikörpern gegen HPV durchgeführt wird, welcher zumindest folgende Schritte umfasst:
Die Probe wird mit einem Reagenz gemischt, welches im Wesentlichen aus einer vorgegebenen Menge an physiologisch wirkender Flüssigkeit und einer vorgegebenen Menge wenigstens eines bezüglich der HPV mit dem Stimulusmolekül spezifischen Antigens besteht;
anschließend wird das Gemisch einer Analyse zugeführt, welche eine messbare und/oder von einem Benutzer wahrnehmbare Veränderung, vorzugsweise gegenüber einem Referenzzustand, nutzt.

Dadurch ist ein Nachweis von körpereigenen Antikörpern gegen HPV mit dem Stimulusmolekül in einfacher Weise und mit wenig Aufwand erbringbar.

Beispielsweise kann hierzu ein Schnelltest zum qualitativen und/oder quantitativen Bestimmen von in Körperflüssigkeit enthaltenen Antikörper gegen HPV angewendet werden, welcher in der deutschen Patentanmeldung mit dem Aktenzeichen 10 2010 061 028.3 beschrieben ist.

Bevorzugt sollte dieser Schnelltest mit dem in der deutschen Patentanmeldung mit dem Aktenzeichen 10 2010 061 028.3 beschriebenen Analysesystem ausgeführt werden, also das dort beschriebene Analysesystem zur Anwendung kommen. Insofern wird auf die deutsche

Patentanmeldung 10 2010 061 028.3 vollumfänglich Bezug genommen und ist vollumfänglich Teil dieser Patentanmeldung.

Durch den Schnelltest ist eine aufwendige und komplizierte Analyse in einem Zentrallabor nicht notwendig. Insofern ist die diagnostische Untersuchung bereits vor Ort, also in der Praxis eines niedergelassenen Arztes oder im Krankenhaus unmittelbar auf der Krankenstation möglich.

Als physiologisch wirkende Flüssigkeit kann im Zuge des Tests beispielsweise eine Kochsalzlösung genutzt werden.

Im Zuge des Testes kommt ein HPV-spezifisches Antigen zum Einsatz, welches Antikörper gegen HPV mit dem Stimulusmolekül identifiziert. Da das Stimulusmolekül ein L1-Protein ist, handelt es sich bei dem HPV-spezifischen Antigen um ein HPVL1-spezifisches Antigen, nämlich um ein HPV16L1-spezifisches Antigen, welches Antikörper gegen das L1-Protein der HPV vom Typ 16 identifiziert.

Sofern in der Probe mittels des Tests die HPV-spezifischen Antikörper gegen HPV mit dem Stimulusmolekül festgestellt werden, handelt es sich um solche, die von dem Immunsystem des Probanden gebildet wurden, also um körpereigene Antikörper.

Durch die Zugabe des HPV-spezifischen Antigens wird bereits vor der Analyse ein Komplex aus dem Antigen und etwaigen in der Probe enthaltenen Antikörpern gegen das HPV mit dem Stimulusmolekül gebildet. Sofern in der Probe die zu dem wenigstens einen Antigen zugehörigen Antikörper enthalten sind, wird das Reagenz inaktiviert. Sofern keine entsprechenden Antikörper in der Probe enthalten sind, bleibt das Reagenz reaktiv. Diese reaktive Eigenschaft des Reagenzes wird dann bei der Analyse genutzt.

Je nachdem, ob in der Probe Antikörper gegen humane Papillomviren mit dem Stimulusmolekül enthalten sind oder nicht, kommt es im Zuge der Analyse zu einer messbaren und/oder von einem Benutzer wahrnehmbaren Veränderung oder es bleibt eine solche Veränderung aus. Bei dem Test wird somit durch die Analyse durch Messung bzw. Wahrnehmung eines Benutzers herausgefunden, ob die zu dem wenigstens einen Antigen zugehörigen Antikörper in der Probe vorhanden sind bzw. oberhalb der Nachweisgrenze des Verfahrens liegen oder nicht vorhanden sind bzw. unterhalb der Nachweisgrenze des Verfahrens liegen.

Die von dem Benutzer wahrnehmbare Veränderung im Zuge der Analyse kann eine optische, akustische und/oder taktile Veränderung, vorzugsweise gegenüber einem Referenzzustand, sein. Beispielsweise kann ein fluoreszierendes Medium genutzt werden. Die Analyse kann einen Färbungseffekt bzw. Verfärbungseffekt oder eine Farbreaktion nutzen. Grundsätzlich kann die Analyse jegliche Art einer messbaren Reaktion nutzen. Durch alle diese Möglichkeiten kann das Analyseergebnis rasch ermittelt werden.

Bevorzugt wird bei fehlenden Antikörpern gegen das Stimulusmolekül oder bei einer Menge von körpereigenen HPV-Antikörpern in der Probe, welche unterhalb oder im Bereich einer vorgegebenen Nachweisgrenze für das HPV-spezifische Antigen liegen, ein Medium, insbesondere über einen vorgegebenen Flächenabschnitt oder ein vorgegebenes Volumen, von einem Benutzer wahrnehmbar und/oder messbar, vorzugsweise gegenüber einem Referenzzustand, verändert. Die Veränderung kann über einen vorgegebenen Flächenabschnitt des Mediums oder über ein vorgegebenes Volumen des Mediums stattfinden.

Es sollte der Analyse eine vorgegebene Menge des Gemisches aus dem Reagenz und der Probe an Körperflüssigkeit zugeführt werden. Bevorzugt sollte die vorgegebene Menge eine Teilmenge des vorhandenen Gemisches sein. Dadurch lassen sich aus der entnommenen Probe und dem damit vereinigten Reagenz mehrere diagnostische Untersuchungen vornehmen, beispielsweise um eine statistische Absicherung des Ergebnisses zu erlangen.

Es kann ferner die Menge des wenigstens einen HPV-spezifischen Antigens durch eine vorgegebene Nachweisgrenze für die zu bestimmende Menge des in der Probe enthaltenen wenigstens einen HPV-Antikörpers bestimmt werden. Die Nachweisgrenze ist bevorzugt die jeweils erwünschte Nachweisgrenze, beispielsweise um zu ermitteln, dass eine bestimmte Menge an Antikörpern gegen HPV mit dem Stimulusmolekül in der Flüssigkeitsprobe enthalten ist, um damit zu bestätigen, dass das Immunsystem auf das Vorhandensein des HPV mit dem Stimulusmolekül bereits angesprungen ist und reagiert hat.

Weitere Ziele, Vorteile, Merkmale und Anwendungsmöglichkeiten der vorliegenden Erfindung ergeben sich aus der nachfolgenden Beschreibung eines Ausführungsbeispieles anhand der Zeichnung.

Es zeigen:
- Fig. 1: eine schematische Darstellung zur Beschreibung einer möglichen Vorgehensweise bei der Durchführung des erfindungsgemäßen Verfahrens, welches zum Überprüfen der Selbstheilung durch das Immunsystem eines mit humanen Papillomviren infizierten Menschen geeignet ist,
- Fig. 2A bis Fig. 2D: Verfahrensschritte bei der Durchführung eines Schnelltests zum qualitativen und/oder quantitativen Bestimmen von in Körperflüssigkeit enthaltenen Antikörpern gegen humane Papillomviren im Zuge der Durchführung eines Verfahrens, wie es der Darstellung in der Figur 1 zugrunde liegt.

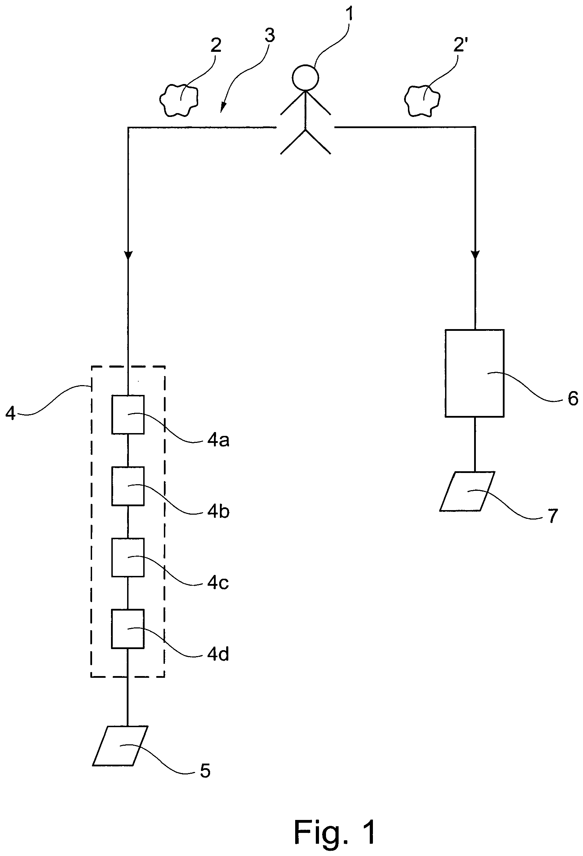

Figur 1 zeigt schematisiert eine mögliche Vorgehensweise bei der Durchführung des erfindungsgemäßen Verfahrens, welches zum Überprüfen der Selbstheilung durch das Immunsystem eines mit humanen Papillomviren (HPV) infizierten Menschen geeignet ist.

Nach dem Verfahren wird in einem ersten Schritt 3 von einem Probanden 1 eine Probe 2 mit Zellen einer Schleimhaut des Probanden 1 entnommen. Beispielsweise kann die Entnahme durch einen Abstrich von der Oberfläche einer Schleimhaut des Probanden 1 erfolgen. Bei der Schleimhaut kann es sich beispielsweise um die Schleimhaut der Gebärmutter einer Frau handeln.

Anhand der Probe 2 wird in einem weiteren Schritt 4 festgestellt, ob HPV mit wenigstens einem das Immunsystem des Probanden stimulierenden HPV-spezifischen Stimulusmoleküls in der Probe 2 enthalten sind. Es wird dazu festgestellt, ob die HPV wenigstens ein L1-Protein als Stimulusmolekül aufweisen.

Sofern im Zuge des Schrittes 4 festgestellt wird, dass als Ergebnis 5 in der Probe 2 HPV mit dem Stimulusmolekül enthalten ist, ist bereits eine wesentliche Voraussetzung gegeben, dass das Immunsystem des Probanden 1 gegen die HPV mit dem Stimulusmolekül vorgeht und die HPV zerstört und somit die Bildung eines HPV-verursachten Karzinoms verhindert.

Es bleibt noch zu überprüfen, ob das Immunsystem des Probanden durch das Stimulusmolekül tatsächlich stimuliert worden ist und somit durch das Stimulusmolekül aktiviert wurde, um gegen die HPV mit dem Stimulusmolekül vorzugehen. Hierzu wird von dem Probanden 1 eine weitere Probe 2' entnommen, mittels welcher dann festgestellt wird, ob der Proband bereits körpereigene Antikörper gegen die HPV mit dem Stimulusmolekül gebildet hat.

Dieser Schritt 6 zur Überprüfung auf das Vorhandensein von körpereigenen Antikörpern gegen die HPV mit dem Stimulusmolekül kann mehrfach wiederholt werden, insbesondere durch mehrfache zeitversetzte Probenahmen 2'. Grundsätzlich ist es aber auch möglich, dass die Überprüfung, ob körpereigene Antikörper gegen die HPV mit dem Stimulusmolekül bereits vorliegen, auch anhand der Probe 2 möglich, anhand welcher bereits der vorhergehende Schritt 4 ausgeführt wurde.

Am Ende der Durchführung des erfindungsgemäßen Verfahrens kann als Ergebnis 7 die Aussage getroffen werden, dass auf die vorhandenen HPV mit dem Stimulusmolekül das Immunsystem des Probanden 1 bereits körpereigene Antikörper gegen die HPV mit dem Stimulusmolekül gebildet hat. In diesem Fall wird die Bildung eines HPV-verursachten Karzinoms mit sehr hoher Wahrscheinlichkeit durch das Immunsystem des Probanden 1 verhindert.

Sofern das Ergebnis 7 lautet, dass das Immunsystem des Probanden 1 auf die HPV mit dem Stimulusmolekül nicht angesprungen ist, also keine körpereigenen Antikörper gegen die HPV mit dem Stimulusmolekül festgestellt werden, wird es mit hoher Wahrscheinlichkeit zur Bildung eines HPV-verursachten Karzinoms kommen.

Im Zuge der Durchführung des Schrittes 4, in welchem festgestellt wird, ob HPV mit dem Stimulusmolekül in der Probe 2 enthalten sind, wird ein erster Unterschritt 4a ausgeführt, in dem ein Erkennen einer morphologischen Veränderung und/oder molekularbiologischen Veränderung wenigstens einer der Zellen erfolgt.

Dazu wird bevorzugt die Probe 2 mit den Zellen auf einen Objektträger, insbesondere Glasobjektträger, gebracht. Anschließend wird bevorzugt ein sogenannter Pap-Test ausgeführt. Dazu werden die in der Probe 2 enthaltenen Zellen mittels eines Farbstoffes angefärbt und die Probe 2 mit den angefärbten Zellen einer mikroskopischen Auswertung unterzogen.

Im Zuge der mikroskopischen Auswertung wird beispielsweise durch einen Benutzer nach morphologischen Veränderungen der Zellen gesucht. Sofern eine solche wenigstens eine morphologische Veränderung wenigstens einer Zelle der Probe festgestellt wird, ist zu überprüfen, ob diese morphologische Veränderung durch HPV, insbesondere HPV mit dem Stimulusmolekül verursacht wurde.

Bevorzugt wird dazu in einem weiteren Unterschritt 4b des Schrittes 4 ein weiterer Test ausgeführt. Im Zuge dieses Tests wird die Probe 2 zuerst einer Antigen-Demaskierung unterzogen. Dazu wird die Probe 2 bei circa 95 Grad Celsius wärmebehandelt.

Anschließend werden der Probe 2 wenigstens einer, vorzugsweise mehrere monoklonale Mausantikörper gleicher Art zugegeben. Der monoklonale Mausantikörper ist bevorzugt darauf ausgerichtet, dass er sich an die wenigstens eine HPV infizierte Zelle mit dem Stimulusmolekül anhaftet. Die Wärmebehandlung und die Zugabe der Mausantikörper erfolgen jeweils vorzugsweise auf den Objektträger mit der Probe 2.

Anschließend erfolgt eine Auswaschung der Mausantikörper. Dazu werden die Mausantikörper von dem Objektträger herausgewaschen, welche noch frei vorhanden sind, also nicht an HPV infizierten Zellen anhaften.

In einem weiteren Schritt 4c werden nun die an den HPV mit dem Stimulusmolekül haftenden Mausantikörper sichtbar gemacht. Bevorzugt wird hierzu ein weiterer Mausantikörper auf die Probe 2 gebracht, welcher sich ebenfalls an die HPV mit dem Stimulusmolekül anhaftet oder zumindest an den an die bereits an dem HPV mit dem Stimulusmolekül anhaftenden vorherigen Mausantikörper anhaftet.

Die weiteren Mausantikörper sind mit wenigstens einem Enzym, wie beispielsweise Peroxidase oder Phosphatase verbunden, welches die Farbneigung des Komplexes aus Mausantikörper und HPV-Stimulusmolekül verstärkt.

In einem weiteren Schritt werden nunmehr die nichthaftenden Bestandteile der zugegebenen Mausantikörper wiederum von der Probe 2 bzw. von dem Objektträger mit der Probe 2 ausgewaschen, so dass auf dem Objektträger mit der Probe 2 ausschließlich Mausantikörper verbleiben, welche an den Zellen mit HPV und dem Stimulusmolekül anhaften.

Anschließend wird nunmehr ein Farbstoff, beispielsweise ein Chromogen, auf die Probe 2, insbesondere den Objektträger mit der Probe 2, gegeben und beispielsweise einer mikroskopischen Untersuchung unterzogen. Durch die Zugabe des Farbstoffes ist unter dem Mikroskop eine Niederschlagsfärbung dort erkennbar, wo das Stimulusmolekül mit dem Mausantikörper und/oder dem weiteren Mausantikörper mit dem damit verbundenen Enzym einen Bindungskomplex gebildet hat. Sofern also derartige Verfärbungen unter dem Mikroskop zu erkennen sind, ist dadurch festgestellt, dass in der Probe 2 HPV mit dem Stimulusmolekül vorhanden sind.

Der Schritt 6 zur Feststellung, ob körpereigene Antikörper gegen die HPV mit dem Stimulusmolekül von dem Probanden gebildet werden, wird bevorzugt mittels eines Schnelltests zum qualitativen und/oder quantitativen Bestimmen von in Körperflüssigkeit enthaltenen Antikörpern gegen HPV durchgeführt, wie er in der deutschen Patentanmeldung 10 2010 061 028.3 beschrieben ist. Ferner kommen dazu bevorzugt ein Analysesystem und eine Vorrichtung zur Durchführung eines solchen Schnelltests zum Einsatz, wie in der deutschen Patentanmeldung 10 2010 061 028.3 ebenfalls beschrieben ist.

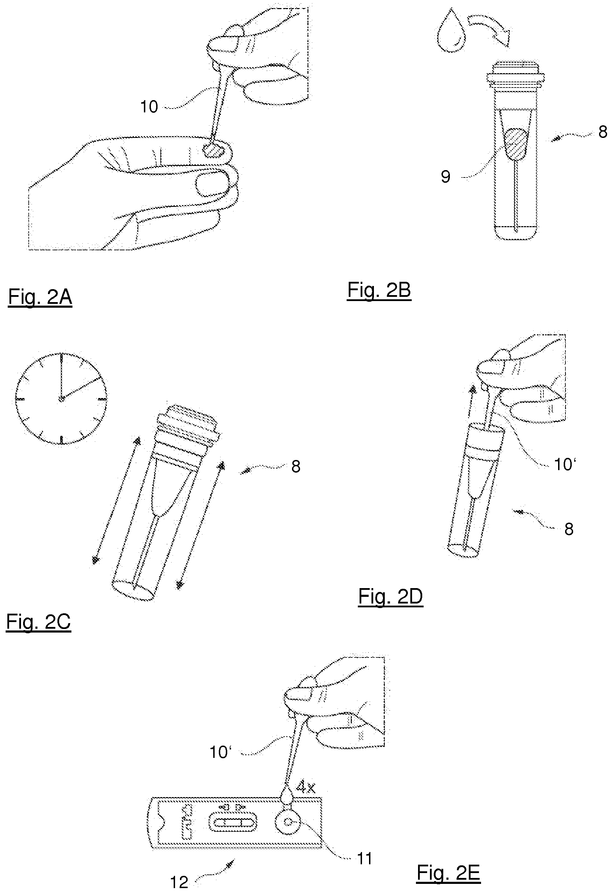

Wie am Beispiel der Figuren 2A bis 2E ersichtlich ist, kann die Probe 2' mittels einer Pipette 10 entnommen werden (Fig. 2A). Dabei kann es sich um eine Probe an Vollblut oder wiederum um eine Probe von anderer Körperflüssigkeit, wie beispielsweise Schleim einer Schleimhautoberfläche des Probanden handeln.

Die Probe 2' wird in ein Behältnis 8 zugegeben, in welcher sich ein Reagenz 9 befindet und mit dem Reagenz 9 vermischt (Fig. 2B). Das Reagenz 9 besteht im Wesentlichen aus einer vorgegebenen Menge an physiologisch wirkender Flüssigkeit, wie beispielsweise Kochsalzlösung und einer vorgegebenen Menge wenigstens eines HPV-spezifischen Antigens.

Vorliegend handelt es sich bei dem HPV-spezifischen Antigen um ein solches Antigen, welches Antikörper gegen HPV mit dem Stimulusmolekül identifiziert und sich an diese derartigen Antikörper anbindet. Bei dem Antigen handelt es sich um ein HPVL1-spezifisches Antigen.

Zur Vermischung der Probe 2' mit dem Reagenz 9 wird das Behältnis 8 mehrfach geschüttelt und über einen vorgegebenen Zeitraum, beispielsweise 10 Minuten, stehen gelassen. Hierdurch hat eine Vermischung der Probenmengen mit dem Reagenz 9 stattgefunden (Figur 2C).

In einem weiteren Schritt wird dann das in dem Behältnis 8 nun befindliche Substrat mittels einer Pipette 10' teilweise entnommen und einer vorgegebenen Menge, beispielsweise einer oder mehrerer Tropfen des Substrates, über eine Befüllstelle 11 einem Analysesystem 12 zugeführt. Das Analysesystem 12 weist ein Konjugat auf, welches im Wesentlichen aus einer vorgegebenen Menge eines Moleküls besteht, welches auf das Stimulusmolekül reagiert, wobei das Konjugat mit koloidalem Gold markiert ist. Das Molekül des Konjugats ist ein L1-spezifisches Molekül, welches ein L1-Protein als Stimulusmolekül identifiziert.

Nach Zugabe des Subtrates zu dem Analysesystem 12 passiert nunmehr folgendes (Fig. 2E): Sofern in der Patientenprobe keine über der Nachweisgrenze des Analysesystems 12 liegende Menge an Antikörpern gegen HPV mit dem Stimulusmolekül vorhanden sind, kommt es zu einer Verfärbung in einem vorgesehenen Bereich des Analysesystems 12. Sofern Antikörper gegen HPV mit dem Stimulusmolekül in der Probe vorhanden sind bzw. über der Nachweisgrenze liegen, bleibt eine solche Verfärbung aus.

### Bezugszeichenliste

- 1: Proband
- 2: Probe
- 2': Probe
- 3: Verfahrensschritt
- 4: Verfahrensschritt
- 4a: Unterschritt
- 4b: Unterschritt
- 4c: Unterschritt
- 4d: Unterschritt
- 5: Ergebnis
- 6: Verfahrensschritt
- 7: Ergebnis
- 8: Behältnis
- 9: Reagenz
- 10: Pipette
- 10': Pipette
- 11: Befüllstelle
- 12: Analysesystem

## Patentansprüche

1. Verfahren zur Vorhersage der Bildung von Karzinomen, welche durch humane Papillomviren (HPV) bedingt sind, bei einem mit den HPV infizierten Probanden, umfassend folgende Schritte:
1) Entnahme wenigstens zweier, Zellen enthaltender Proben von dem Probanden, wobei eine der wenigstens zwei Proben eine Probe von Schleimhaut (Schleimhautprobe) und eine andere der wenigstens zwei Proben eine Probe von Körperflüssigkeit (Körperflüssigkeitsprobe) ist;
2) Feststellung, ob in der Schleimhautprobe die HPV wenigstens eine das Immunsystem des Probanden stimulierendes Stimulusmolekül enthalten, indem mittels Antikörper eine morphologische Veränderung und/oder eine molekularbiologische Veränderung wenigstens einer der Zellen der Schleimhautprobe als HPV-verursachte Veränderung erkannt wird, an welcher die HPV mit dem Stimulusmolekül beteiligt sind, wobei das betrachtete Stimulusmolekül das L1-Protein des Kapsids der HPV vom Typ 16 (HPV16) ist und die Antikörper gegen das Stimulusmolekül spezifisch sind;
3) Feststellung, ob in der Körperflüssigkeitsprobe körpereigene Antikörper gegen die HPV mit dem Stimulusmolekül enthalten sind, indem die Körperflüssigkeitsprobe mit einem Reagenz gemischt wird, welches im Wesentlichen aus einer vorgegebenen Menge an physiologisch wirkender Flüssigkeit und einer vorgegebenen Menge wenigstens eines Antigens besteht, und anschließend das Gemisch einer Analyse zugeführt wird, welche eine messbare und/oder von einem Benutzer wahrnehmbare Veränderung nutzt, wobei das Antigen bezüglich in der Körperflüssigkeitsprobe enthaltener Antikörper reaktiv ist, welche gegen das Stimulusmolekül der HPV16 spezifisch sind (HPV16L1 spezifische Antikörper);
4) Auswertung dahingehend, dass bei Feststellung des Stimulusmoleküls in Schritt (2) und bei Feststellung der körpereigenen Antikörper in Schritt (3) vorhergesagt wird, dass die Bildung von Karzinomen, welche durch die betrachteten HPV bedingt sind, durch das Immunsystem des Probanden mit sehr hoher Wahrscheinlichkeit verhindert ist.

2. Verfahren nach Anspruch 1, bei dem in Schritt (2) zum Erkennen der morphologischen Veränderung der wenigstens einen Zelle ein Pap-Test durchgeführt wird.

3. Verfahren nach Anspruch 1 oder 2, bei dem in Schritt (2) die Schleimhautprobe zuerst wärmebehandelt wird, dann die Antikörper zugegeben werden und anschließend die Schleimhautprobe und die Antikörper einer Analyse zugeführt werden, welche eine messbare und/oder von einem Benutzer wahrnehmbare Veränderung der mit den HPV infizierten Zellen aufgrund der Zugabe der Antikörper nutzt.

4. Verfahren nach Anspruch 3, bei dem in Schritt (2) ein Verstärkermittel zugegeben wird, durch welches bei der Analyse die messbare und/oder von einem Benutzer wahrnehmbare Veränderung verstärkt wird.

5. Verfahren nach einem der vorhergehenden Ansprüche, bei dem in Schritt (3) eine vorgegebene Menge des Gemisches der Analyse zugeführt wird.

6. Verfahren nach einem der vorhergehenden Ansprüche, bei dem in Schritt (3) bei fehlenden HPV16L1 spezifischen Antikörpern in der Körperflüssigkeitsprobe oder bei einer Menge von HPV16L1 spezifischen Antikörpern in der Körperflüssigkeitsprobe, welche unterhalb oder im Bereich einer vorgegebenen Nachweisgrenze für das Antigen liegen, ein Medium, insbesondere über einen vorgegebenen Flächenabschnitt oder ein vorgegebenes Volumen, von einem Benutzer wahrnehmbar und/oder messbar verändert wird.

## Claims

1. Method for predicting the development of cancers caused by human papilloma viruses (HPVs) in a test subject infected with the HPVs, comprising the following steps:
1) collecting at least two cell-containing samples from the test subject, wherein one of the at least two samples is a sample of mucosa (mucosal sample) and the other of the at least two samples is a sample of body fluid (body-fluid sample);
2) establishing whether, in the mucosal sample, the HPVs contain at least one stimulus molecule which stimulates the immune system of the test subject, by using antibodies to identify a morphological change and/or a molecular-biology change of at least one of the cells of the mucosal sample as an HPV-caused change in which the HPVs containing the stimulus molecule are involved, wherein the stimulus molecule contemplated is the L1 protein of the capsid of the HPVs of type 16 (HPV16) and the antibodies are specific against the stimulus molecule;
3) establishing whether the body-fluid sample contains endogenous antibodies against the HPVs containing the stimulus molecule, by mixing the body-fluid sample with a reagent which substantially consists of a predefined amount of physiologically effective liquid and a predefined amount of at least one antigen and subsequently supplying the mixture to an analysis which uses a measurable and/or user-perceivable change, wherein the antigen is reactive with regard to antibodies which are present in the body-fluid sample and which are specific against the HPV16 stimulus molecule (HPV16L1-specific antibodies);
4) making an evaluation to the effect that, in the event of establishing the stimulus molecule in step (2) and in the event of establishing the endogenous antibodies in step (3), it is predicted that the development of cancers caused by the HPVs contemplated is prevented with very high probability by the immune system of the test subject.

2. Method according to Claim 1, in which, in step (2), a Pap test is carried out in order to identify the morphological change of the at least one cell.

3. Method according to Claim 1 or 2, in which, in step (2), the mucosal sample is first heat-treated, the antibodies are then added, and the mucosal sample and the antibodies are subsequently supplied to an analysis which uses a measurable and/or user-perceivable change of the cells infected with the HPVs on the basis of the addition of the antibodies.

4. Method according to Claim 3, in which, in step (2), an enhancer is added, which enhances the measurable and/or user-perceivable change in the analysis.

5. Method according to any of the preceding claims, in which, in step (3), a predefined amount of the mixture is supplied to the analysis.

6. Method according to any of the preceding claims, in which, in step (3), in the event of absent HPV16L1-specific antibodies in the body-fluid sample or in the event of an amount of HPV16L1-specific antibodies in the body-fluid sample which are below or within the region of a predefined detection limit for the antigen, a medium is changed in a user-perceivable manner and/or in a measurable manner, especially over a predefined areal section or a predefined volume.

## Revendications

1. Procédé de prévision de la formation de carcinomes qui sont dus au virus du papillome humain (HPV) chez un sujet participant infecté par le HPV, comprenant les étapes suivantes :
1) prélèvement d'au moins deux échantillons contenant des cellules auprès du sujet participant, l'un des au moins deux échantillons étant un échantillon de muqueuse (échantillon de muqueuse) et un autre des au moins deux échantillons étant un échantillon de fluide corporel (échantillon de fluide corporel) ;
2) détermination si dans l'échantillon de muqueuse, les HPV contiennent au moins une molécule de stimulation qui stimule le système immunitaire du sujet participant en reconnaissant, au moyen d'anticorps, une modification morphologique et/ou une modification biologique moléculaire d'au moins l'une des cellules de l'échantillon de muqueuse en tant que modification causée par le HPV à laquelle participent les HPV avec la molécule de stimulation, la molécule de stimulation observée étant la protéine L1 de la capside du HPV de type 16 (HPV16) et les anticorps étant spécifiques contre la molécule de stimulation ;
3) détermination si dans l'échantillon de fluide corporel se trouvent des anticorps propres au corps contre les HPV avec la molécule de stimulation en mélangeant l'échantillon de fluide corporel avec un réactif qui se compose pour l'essentiel d'une quantité prédéfinie de liquide à action physiologique et d'une quantité prédéfinie d'au moins un antigène, et ensuite en fournissant le mélange à une analyse qui utilise une modification mesurable et/ou perceptible par un utilisateur, l'antigène étant réactif en référence aux anticorps contenus dans l'échantillon de fluide corporel qui sont spécifiques contre la molécule de stimulation du HPV16 (anticorps spécifique au HPV16L1) ;
4) interprétation dans le sens qu'en cas de détermination de la présence de la molécule de stimulation à l'étape (2) et en cas de détermination de la présence des anticorps propres au corps à l'étape (3), il est prédit que la formation de carcinomes qui sont dus aux HPV considérés est empêchée avec une très forte probabilité par le système immunitaire du sujet participant.

2. Procédé selon la revendication 1, avec lequel, à l'étape (2), un test Pap est effectué en vue de reconnaître la modification morphologique de l'au moins une cellule.

3. Procédé selon la revendication 1 ou 2, avec lequel, à l'étape (2), l'échantillon de muqueuse est tout d'abord traité à chaud, après quoi les anticorps sont ajoutés et ensuite l'échantillon de muqueuse et les anticorps sont fournis à une analyse qui utilise une modification mesurable et/ou perceptible par un utilisateur des cellules infectées par des HPV en raison de l'ajout des anticorps.

4. Procédé selon la revendication 3, avec lequel, à l'étape (2), un agent amplificateur est ajouté, par lequel la modification mesurable et/ou perceptible par un utilisateur lors de l'analyse est amplifiée.

5. Procédé selon l'une des revendications précédentes, avec lequel, à l'étape (3), une quantité prédéfinie du mélange est fournie à l'analyse.

6. Procédé selon l'une des revendications précédentes, avec lequel, à l'étape (3), en l'absence d'anticorps spécifiques au HPV16L1 dans l'échantillon de fluide corporel ou en présence d'une quatité d'anticorps spécifiques au HPV16L1 dans l'échantillon de fluide corporel, lesquels se trouvent au-dessous ou dans la plage d'une limite de décèlement prédéfinie pour l'antigène, un milieu est modifié de manière perceptible par un utilisateur et/ou mesurable, notamment sur une portion de surface prédéfinie ou un volume prédéfini.
